# EUROPEAN PATENT SPECIFICATION

(11) **EP 1 598 049 B1**
(45) Date of publication and mention of the grant of the patent: **06.08.2008**
(21) Application number: 04012178.2
(22) Date of filing: 22.05.2004
(51) Int. Cl.: A61K 8/41, A61K 8/49, A61Q 5/10

(54) **Composition for coloring of keratin fibres**
Farbemittel für Keratinfasern
Composition de teinture des fibres kératiniques.

(43) Date of publication of application: 23.11.2005
(73) Proprietor: KPSS-Kao Professional Salon Services GmbH, 64297 Darmstadt (DE)
(72) Inventor: Wood, Jonathan, Dr., 69469 Weinheim (DE); Nöcker, Bernd, Dr., 64372 Ober-Ramstadt (DE); Wilz, Rüdiger, 64319 Pfungstadt (DE)

(56) References cited:
- WO-A-02/074270
- US-A1- 2003 159 221

## Description

The present invention concerns a composition for the dyeing keratin fibers especially human hair on the basis of an oxidation dyestuff precursor system reacting with peroxide and comprising additionally cationic and anionic direct dyes which provides long-lasting, intensive colors either used as such, or which can be used to obtain further shades in combination with additional developing and/or coupling agents.

The developing substances still most frequently used in hair dyeing compositions are 1,4-diaminobenzene (p-phenylenediamine) and 1-methyl-2,5-diaminobenzene (p-toluylenediamine). Although incorporation of these substances largely fulfills the user's color wishes, there are still shades that cannot be completely achieved by the use thereof or which can be intensified. Usually direct dyes of cationic and/or neutral character are used for obtaining further shades. For example, EP 850 636, EP 850 637 and EP 852 135 disclose composition based on oxidative dye precursors and cationic direct dyes. However the colors so obtained are not long last as the cationic dyes are easily washed out by subsequent shampooing and other environmental influences.

US 2003/159221 A1 discloses hair dye compoistions with p-phenylendiamine derivatives and a basic dye which is different from the ones claimed with the present invention. Furthermore D1 does not disclose hair dyeing compositions comprising anionic direct dye.

WO 02/074270 A1 discloses hair dye compositions comprising basic and acidic dye but not comprising an oxidative dye precursor.

The invention therefore starts from the problem of creating a hair dyeing composition suited for the preparation of a large number of shades with especially intensive, glossy appearance and being excellently durable.

This problem is solved when such a hair dyeing composition comprises
a- at least one oxidation dyestuff precursor reacting with peroxide, which is selected from 4-aminophenol and the derivatives thereof of the general formula (I) 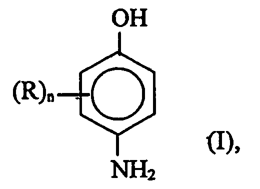 wherein R is a C₁-C₃-alkyl group, a hydroxy-C₁-C₃-alkyl group or a halogen atom, in particular Cl, and n is a number from 0 to 2, and/or 2-aminophenol and/or 1,4 diaminobenzene and substituted p-phenylenediamines and the derivatives thereof of the general formula II 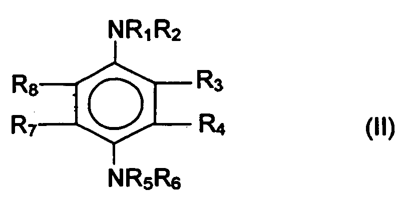 wherein R₁ and R₂ are same or different, and stands for hydrogen, C₁-C₄ alkyl, C₁-C₄ hydroxyalkyl, isopropyl, phenyl or p-aminophenyl, R₃ is hydrogen, C₁-C₄ alkyl, C₁-C₄ hydroxyalkyl, C₁-C₄ alkoxy, chloride or isopropyl, R₄ is hydrogen, halogen, in particular chloride, C₁-C₄ alkyl or C₁-C₄ hydroxyalkyl, R₅ and R₆ are the same or different, a group selected from hydrogen, C1-C4 alkyl, C1 -C4 hydroxyalkyl or isopropyl, R₇ is hydrogen and R₈ is a group selected from hydrogen, hydroxyl, C₁-C₄ alkyl, C₁-C₄ hydroxyalkyl or isopropyl,
b- at least one acidic direct dye, and
c- at least one cationic direct dye selected from the compounds presented with the general formulas III, IV, V and VI 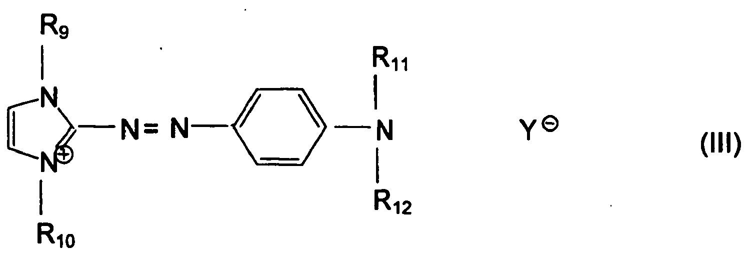 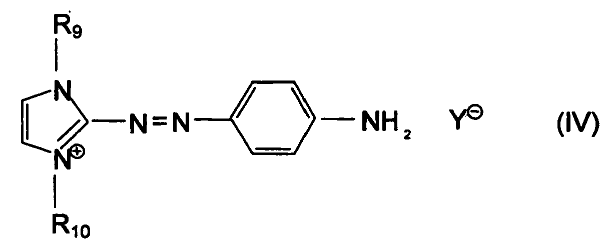 and 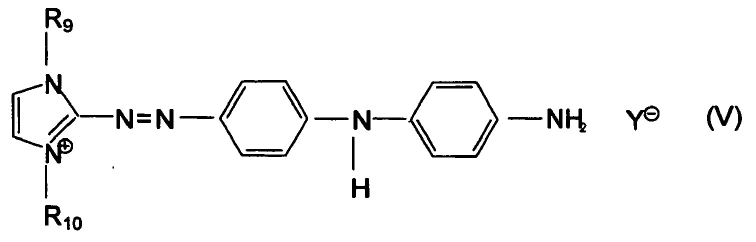
wherein R₉, R₁₀, R₁₁ and R₁₂ stand for hydrogen, a CH₃- or C₂H₅- group, R₁₃ stands for hydrogen, -OCH₃ or -OC₂H₅ and Y is an anion such as chloride, bromide, methosulfate.

Oxidation dyestuff precursors preferred according to formula I above are 4-aminophenol, 4-amino-3-methylphenol, 2-chloro-4-aminophenol, 2,6-dichloro-4-aminophenol, 2,4-diamino-phenol, 2,6-dibromo-4-aminophenol and/or 2-aminophenol and water-soluble salts thereof.

Oxidation dyestuff precursors preferred according to formula II above are in particular 2,5-diamino-toluene, 2-n-propyl or 2-ethyl-p-phenylene-diamine, 2,6-dimethyl-p-phenylenediamine, 2-(2,5-diaminophenyl) ethanol, 1-amino -4-bis-(2'-hydroxyethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-0-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1.3-dimethyl-2,5-diaminobenzene, 1.4-diamino isopropyl benzene and/or 1-amino-4-β-hydroxypropyl aminobenzene or the water-soluble salts thereof.

According to the invention the suitable direct acting anionic dyes are:

Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the most preferred anionic dyestuffs are Acid Red 52, DC Violet 2, DC Red 33, DC Orange 4, DC Red 27 and DC Yellow 10.

According to the invention, suitable cationic dyestuffs are in principal those available on the market for cosmetic hair colouring applications. For this purpose, special reference is made to the PCT application WO 95/15144 of Ciba-Geigy AG.
The most preferred cationic dyes in the colouring compositions of the present invention are the ones according to the formula III, where R₉, R₁₀, R₁₁ and R₁₂ are methyl and Y is chloride, according to formula IV where R₉, and R₁₀ are methyl and Y is chloride and according to the formula VI, where R₉ and R₁₀ are methyl, R₁₃ is hydrogen and Y is methosulfate.

The total concentration of the oxidation dyestuff precursors according to the formula I and/or II customarily ranges between about 0.05 % and 5 %, preferably 0.1 % and 4 %, in particular 0.1 % to 3% by weight, calculated to the total hair dyeing composition (excluding the oxidation agent), whereby these figures are always related to the proportion of free base.

According to the invention, coloring composition comprises anionic dyes at a concentration of 0.1 to 7.5%, preferably 0.2 to 5%, more preferably 0.2 to 3% by weight calculated to total composition. Cationic dyestuffs are included into the compositions of the present invention at a concentration of 0.01 to 2.5%, preferably 0.05 to 2% and more preferably 0.05 to 1.5% by weight calculated to total composition.

Interestingly it has been found out that the ratio of acidic dyes to cationic dyes plays an important role for achieving especially resistance to washing and environmental effects. Accordingly, the ratio of cationic dyestuffs to acidic dyestuffs by weight is in the range of 3:1 to 1:10, preferably 2: 1 to 1:7 and further more preferably 2:1 to 1:5.

The composition according to the invention preferably comprises at least one coupling substance, which can be selected from resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxypyridine, 2-dimethyl-amino-5-aminopyridine, 2,6-diaminopyridine, 1,3-diaminobenzene, 1-amino-3-(2'-hy-droxyethylamino)benzene, 1-amino-3-[bis(2'-hydroxyethyl) amino]benzene, α-naphthol, 4,6-dichlororesorcinol, 1,3-diaminotoluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof. However, this shall not exclude the addition of further developing and coupling substances.

The weight proportion of the named developing substances to the additional developing and coupling substances ranges between about 1 : 8 to 8 : 1, preferably about 1 : 5 to 5 : 1, in particular 1 : 2 to 2 : 1. In the hair dyeing compositions according to the invention, the coupling substance(s) as reaction partners of the developing substance(s) are present in approximately the same molecular proportions as the developing substances, i.e. in amounts from 0.01 % to 5.0 %, preferably 0.05 % to 4 %, in particular 0.1 % to 3 % by weight, calculated to the total composition (excluding the oxidizing agent), whereby these figures are always related to the proportion of free base.

After oxidation with peroxide, use of these compositions on the basis of a customary carrier provides very expressive, intensive, long-lasting hair colorations, which can be varied to achieve further shades by the addition of the respective further developing and coupling substances.

It is also possible to incorporate additional developing substances known **per se**. pyrazole or triazole derivatives such as 1-hydroxyethyl-4.5-diaminopyrazole, 3.4-diamino-5-hydroxypyrazole, 3.5-diaminopyrazole, 3.5-diamino pyrazol-1-carboxamide, 3-amino-5-hydroxypyrazole, 1-phenyl-2-methylpyrazole, 1-phenyl-3-methylpyrazole-5-one, 3.5-dimethylpyrazole, 3.5-dimethylpyrazole-1-methanol, 3.5-diamino-1.2.4-triazole, tetraaminopyrimidine, triaminohydroxypyrimidine, diamino mono- and dihydroxy- pyrimidine, aminotriazines, 5-amino-salicylic acid and/or 1.2.4-triaminobenzene and the water-soluble salts thereof.

Additionally other cationic dyestuffs can as well be used in addition to the cationic dyestuffs mentioned above. Some examples to those are:

Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

Additionally, the coloring compositions of the present invention may comprise neutral dyes (HC dyes), so called nitro dyes for shading purposes. Concentration of those can typically be in the range from 0.01 to 2.5%, preferably 0.1 to 2% by weight calculated to total composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No. 13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

Hair dyeing composition of the present invention preferably comprise an organopolysiloxane wherein at least one silicium atom is linked to an alkylene group having a hetero-atom, in particular a nitrogen atom, with a poly-(N-acyl alkyleneimine) units of the formula 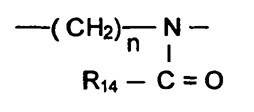 wherein n is a number from 1 to 5 and R₁₄ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 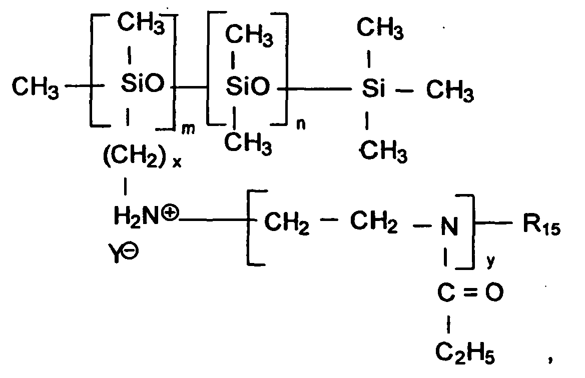 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y- is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Another preferred compound in the colouring composition is of ceramide type of compounds according to general formula 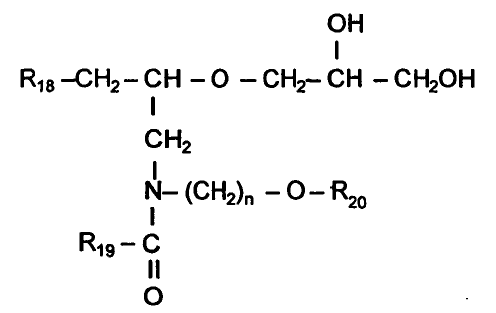 where R₁₈ and R₁₉ are independent from each other alkyl- or. alkenyl group mit 10 to 22 carbon atoms, R₂₀ is methyl, ethyl, n-propyl or isopropyl group and n is a number between 1 to 6, preferably 2 or 3. The concentration of ceramide type of compound in colouring compositions of the present invention can be in the range of 0.01 to 2 and especially 0.01 to 1% by weight calculated to the total composition.

The compositions of the present invention can comprise one or more ubiquinone of the formula. 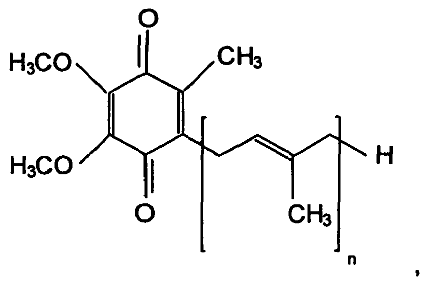 wherein n is a number from 1 to 10. Concentration of ubichinones in the compositions of the present invention can vary between 0.001 % and 10 % by weight, calculated to the total composition excluding oxidizing agent.

Coloring composition of the present invention can certainly comprise compounds for accelerating (catalysts) the oxidative dyeing keratin fibres such as iodine salts i.e. potassium or sodium iodide and/or dihydroxy acetone.

The hair dyeing compositions according to the invention can comprise the basic substances and additives customarily found in such compositions, conditioning agents, etc., known as state of the art and described, for example, in the monography of K. Schrader, "Grundlagen und Rezepturen der Kosmetika", 2nd Ed. (Hüthig Buch Verlag, Heidelberg, 1989), pp. 782 to 815. They can be prepared as solutions, creams, gels or also in the form of aerosol products; suitable carrier material compositions are known as state of the art.

For application, the oxidation dyestuff precursor is mixed with an oxidizing agent. The preferred oxidizing agent is hydrogen peroxide, for example in a concentration of 2 to 12 % by weight. However, the use of other peroxides such as urea peroxide and melamine peroxide is also possible.

As an alternative to peroxide oxidation, it is also possible to achieve the oxidation by air, for example, by applying onto the hair a composition comprising an oxidation dyestuff precursor as aerosol foam and leaving to process for about 15 to 20 minutes.

The pH-value of the ready-to-use hair dyeing composition, i.e. after mixing with peroxide, can be between 5 and 12, preferably 6 - 11, more preferably 6.8 to 10.

The following examples are to illustrate the invention without limiting it.

| Carrier | |
|---|---|
| Stearyl alcohol | 8.0 (% by wt.) |
| Coco fatty acid monoethanolamide | 4.5 |
| 1.2-Propanediol mono/distearate | 1.3 |
| Coco fatty alcohol polyglycolether | 4.0 |
| Sodium lauryl sulfate | 1.0 |
| Oleic acid | 2.0 |
| 1.2-Propanediol | 1.5 |
| Na-EDTA | 0.5 |
| Sodium sulfite | 1.0 |
| Protein hydrolyzate | 0.5 |
| Ceramide according to formerly stated formula where | 0.2 |
| R₁₈ and R₁₉ are C16 and R₂₀ is ethyl | |
| Ascorbic acid | 0.2 |
| Organopolisiloxane according to EP6406430.3 | |
| "Compound A-1" | |
| Perfume | 0.4 |
| Ubichinone 10 | 0.1 |
| Ammonia, 25% | 1.0 |
| Ammonium chloride | 0.5 |
| Panthenol | 0.8 |
| Water | ad 100.00 |

The dyestuff combinations either oxidative or direct dyes of anionic and cationic characters were incorporated into this carrier, whereby the water content was reduced accordingly.

The colorations were carried out on wool patches, strands of bleached human hair and natural human hair at a color level of 8 by application of a 1:1 mixture of an emulsion comprising the dyestuffs as given in the examples below and a 6 % hydrogen peroxide solution (pH-value of the mixture: 9.8) with 30 minutes processing at room temperature, subsequent rinsing and drying.

The following colorations were obtained:

**Table I: Examples 1 to 5**

| | Example | | | | |
|---|---|---|---|---|---|
| | I | II | III | IV | V |
| | (all values are weight %) | | | | |
| p-phenylenediamine sulfate | 0.90 | 0.90 | 0.90 | 0.90 | 0.90 |
| a-naphtol | 0.63 | 0.63 | 0.63 | 0.63 | 0.63 |
| cationic direct dye of formula (III) with R₉=R₁₀=R₁₁=R₁₂= methyl and Y is chloride | 0.06 | 0.04 | 0.02 | 0.08 | - |
| Acid red 52 | 0.02 | 0.04 | 0.06 | - | 0.08 |

Examples I to III are according to the invention and examples IV and V are for comparative purposes.

The coloring compositions so obtained show excellent dyeing performance.

In order show color fastness (durability) against washing, all colored strands were washed 10 times under usual hair wash conditions with a commercial shampoo composition designed for colored hair of the trade mark Goldwell Definition, and color of the tresses (L, a and b values) were measured before and after shampooing optically with a laboratory equipment and color differences were calculated with the well known equation to obtain ΔE values and color intensity differences (ΔL) were obtained from measured L values. The results are presented in the Table II below.

**Table II: Results of the durability test**

| Example | L1 | L2 | ΔE |
|---|---|---|---|
| I | 27.2 | 29 | 9.0 |
| II | 27.6 | 29.2 | 8.0 |
| III | 28.9 | 31.1 | 5.7 |
| IV | 28.3 | 34 | 14 |
| V | 29.0 | 32 | 12 |

L1 stands for the intensity of the color measured before the washing test and L2 is the same value measured after washing the strands 10 times with shampoo. ΔE value is obtained from the L, a and b values measured before and after washing. As it is obvious from the table immediately after coloring there is no real difference in the intensity though shade differences were obvious (not shown). However, after washing the strands with shampoo, intensity differences were obvious between the strands colored only with either cationic or anionic dyes (non inventive examples IV and V, respectively) and the strands colored with mixture of anionic and cationic dyes according to the present invention. This is furthermore expressed with ΔE values as the color difference.

## Claims

1. Composition for the dyeing of keratin fibers, especially human hair, **characterized**
**in that** it comprises
a- at least one oxidation dyestuff precursor, selected from 4-aminophenol and the derivatives thereof of the general formula (I) 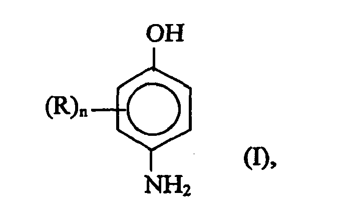 wherein R is a C₁-C₃-alkyl group, a hydroxy-C₁-C₃-alkyl group or a halogen atom, in particular Cl, and n is a number from 0 to 2, or 2-aminophenol or 1,4 diaminobenzene or substituted p-phenylenediamines and the derivatives thereof of the general formula II 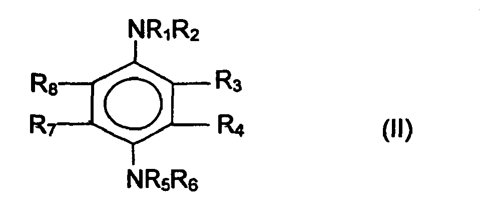 wherein R₁ and R₂ are same or different, and stands for hydrogen, C₁-C₄ alkyl, C₁-C₄ hydroxyalkyl, isopropyl, phenyl or p-aminophenyl, R₃ is hydrogen, C₁-C₄ alkyl, C₁-C₄ hydroxyalkyl, C₁-C₄ alkoxy, chloride or isopropyl, R₄ is hydrogen, halogen, in particular chloride, C₁-C₄ alkyl or C₁-C₄ hydroxyalkyl, R₅ and R₆ are the same or different, a group selected from hydrogen, C₁-C₄ alkyl, C₁ -C₄ hydroxyalkyl or isopropyl, R₇ is hydrogen and R₈ is a group selected from hydrogen, hydroxyl, C₁-C₄ alkyl, C₁-C₄ hydroxyalkyl or isopropyl,
b- at least one acidic direct dye, and
c- at least one cationic direct dye selected from the compounds presented with the general formulas III, IV, V and VI 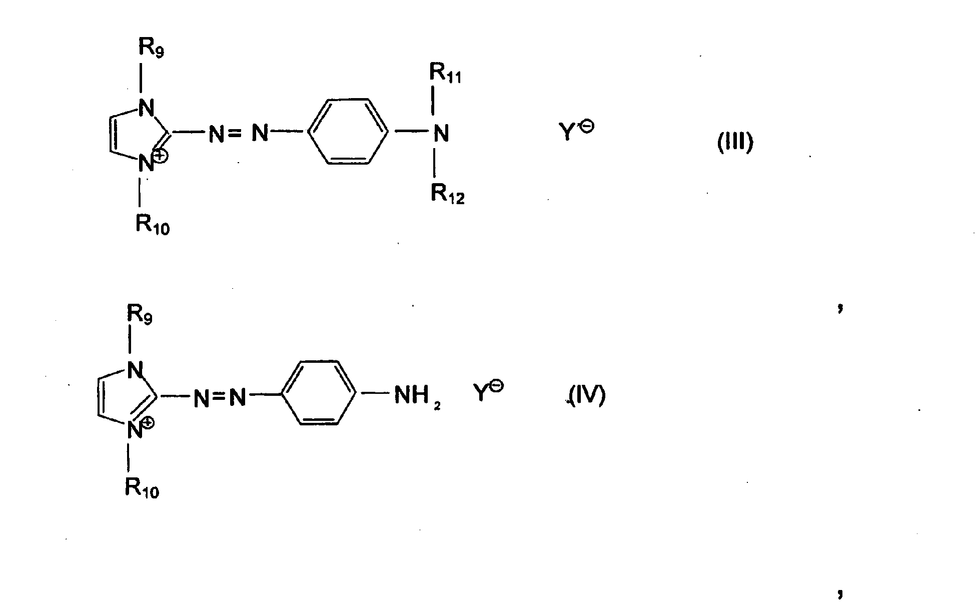 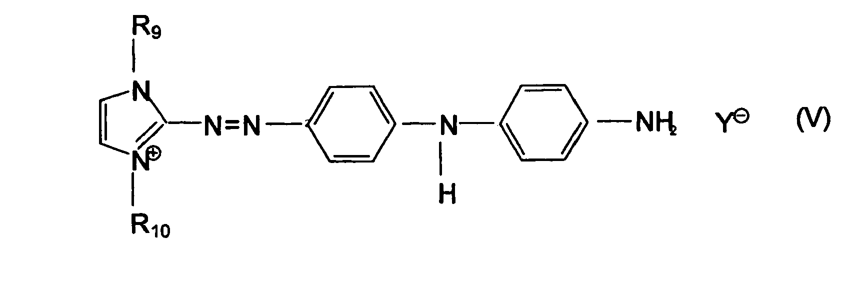 and 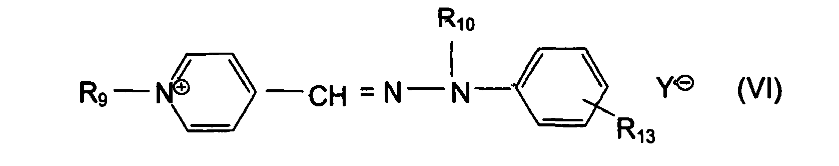
wherein R₉, R₁₀, R₁₁ and R₁₂ stand for hydrogen, a CH₃- or C₂H₅- group, R₁₃ stands for hydrogen, -OCH₃ or -OC₂H₅ and Y is an anion such as chloride, bromide, methosulfate.

2. Composition according to claim 1 **characterized in that** it comprises as the component a at least one oxidation dyestuff precursor selected from the compounds 4-aminophenol, 4-amino-3-methylphenol, 2-chloro-4-aminophenol, 2.6-dichloro-4-aminophenol, 2.4-diaminophenol, 2,6-dibromo-4-aminophenol and/or 2-aminophenol.

3. Composition according to claim 1, **characterized in that** it comprises as the component a at least one oxidation dyestuff precursor -selected from the compounds 2,5-diamino-toluene, 2-n-propyl or 2-ethyl-p-phenylene-diamine, 2,6-dimethyl-p-phenylenediamine, 2-(2,5-diaminophenyl) ethanol, 1-amino -4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenyleruediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyt aminobenzene, 1-dimethyl-amino-4-aminobenzene, 1-hydroxy-2,5-diamino-4-methyl benzene, 1-hydroxymethyl-2,5-diaminobenzene, 1.3-dimethyl-2,5-diaminobenzene, 1.4-diamino isopropyl benzene and/or 1-amino-4-β-hydroxypropy) aminobenzene or the water-soluble salts thereof.

4. Composition according to any of the preceding claims **characterized in that** it comprises as the component b at least one acidic anionic dyestuff selected from the compounds Acid Red 52, DC Violet 2, DC Red 33, DC Orange 4, DC Red 27 and DC Yellow 10.

5. Composition according to any of the preceding claims **characterized in that** it comprises as the component c the cationic dyestuff according to formula III 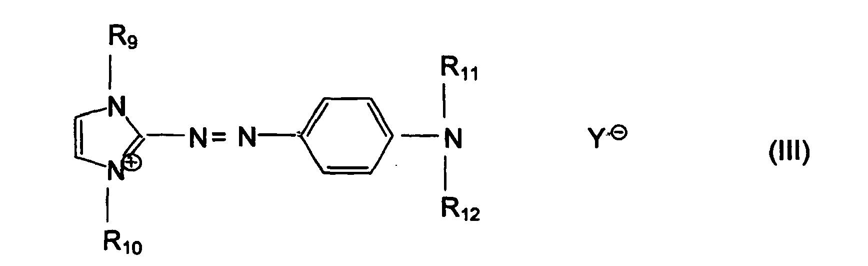 wherein R₉, R₁₀, R₁₁ and R₁₂ are methyl and Y is chloride.

6. Composition according to claims 1 to 4 **characterized in that** it comprises as the component c the cationic dyestuff according to formula IV 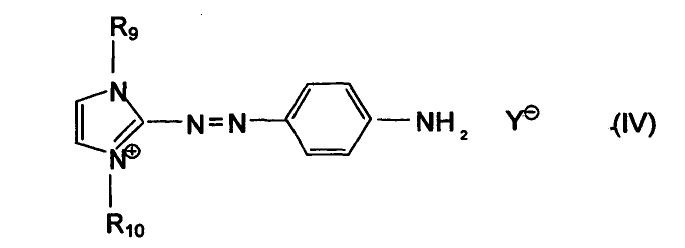 wherein R₉, and R₁₀ are methyl and Y is chloride.

7. Composition according to claims 1 to 4 **characterized in that** it comprises as the component c the cationic dyestuff according to formula VI 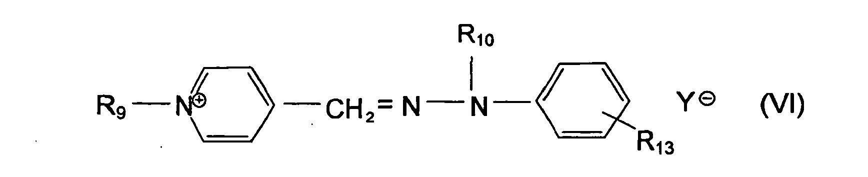 wherein R₉ and R₁₀ are methyl, R₁₃ is hydrogen and Y is methosulfate.

8. Composition according to any of the preceding claims **characterized in that** it comprises component a at a concentration of 0.01 to 5% by weight calculated to total composition excluding oxidizing agent.

9. Composition according to any of the preceding claims **characterized in that** it comprises component b at a concentration of 0.1 to 7.5% by weight calculated to total composition excluding oxidizing agent.

10. Composition according to any of the preceding claims **characterized in that** it comprises component c at a concentration of 0.01 to 2.5% by weight calculated to total composition excluding oxidizing agent.

11. Composition according to any of the preceding claims **characterized in that** it comprises components b and c at a weight ratio of (cationic dyestuff : anionic dyestuff) 3:1 to 1:10, preferably 2:1 to 1:7.

12. Composition according to any of the preceding claims **characterised in that** it comprises additionally at least one coupling substance selected from resorcinol,-2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2.6-diaminopyridine, 1.3-diaminobenzene, 1-amino-3-(2'-hydroxyethylamino)benzene, 1-amino-3-[bis(2'-hydroxy-ethyl) amino]benzene, α-naphthol, 4.6-dichlororesorcinol, 1.3-diaminotoluene, 1-hydroxy naphthalene, 4-hydroxy-1.2-methy-lenedioxy benzene, 1.5-dihydroxy naphthalene, 1.6-dihydroxy naphthalene, 1.7-dihydroxy naphthalene, 2.7-dihydroxy naphthalene, 1-hydroxy-2-methyl naphthalene, 4-hydroxy-1.2-methyldioxy benzene, 2.4-diamino-3-chlorophenol and/or 1-methoxy-2-amino-4-(2'-hydroxyethyl amino)benzene -or the water-soluble salts thereof.

13. Composition according to any of the preceding claims **characterized in that** it comprises organosiloxane polymer according to formula 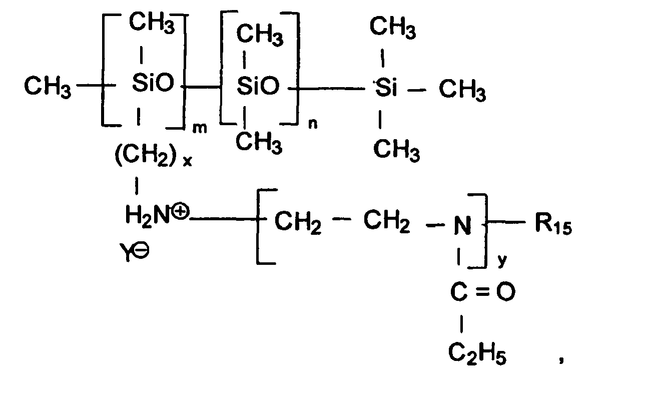 wherein m and n each are numbers from 20 to 10,000, x is a number between 1 and 5, and y is a number from 5 to 30, R₁₅ is a C₁-C₁₂-alkyl or aryl group, in particular a methyl, ethyl or benzyl group, and Y⁻ is an anion.

14. Composition according to any of the preceding claims **characterized in that** it comprises additionally at least one ceramide type compound according to the formula 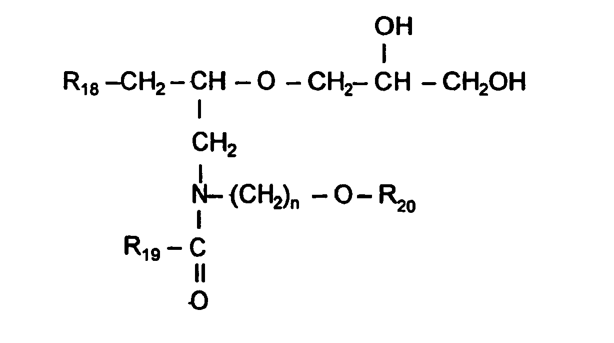 where R₁₈ and R₁₉ are independent from each other alkyl- or. alkenyl group mit 10 to 22 carbon atoms, R₂₀ is methyl, ethyl, n-propyl or isopropyl group and n is a number between 1 to 6, preferably 2 or 3.

15. Composition according to any of the preceding caharactersed in that it comprises of at least one ubiquinone of the formula 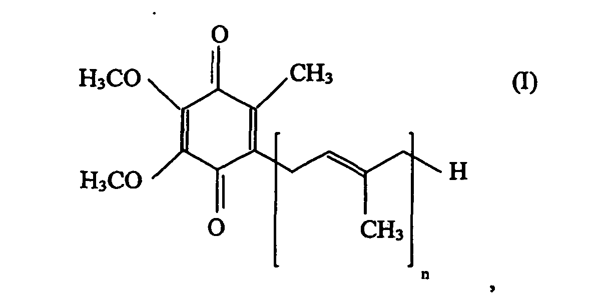 wherein n is a number from 1 to 10.

16. Composition according to any of the preceding claims **characterized in that** it comprises additional cationic and/or neutral HC dyes.

17. Composition according to any of the preceding claims **characterized in that** it comprises additionally potassium or sodium iodide and/or dihydroxyacetone for as catalysts.

18. Use of compositions according to any of the preceding claims for durable coloring keratin fibers, especially hair.

## Patentansprüche

1. Zusammensetzung zum Färben von Keratin-Fasern, besonders menschlichen Haaren, **dadurch gekennzeichnet, dass** sie
a- mindestens einen Oxydationsfarbstoff-Entwickler, ausgewählt aus 4-Aminophenol und dessen Derivaten mit der allgemeinen Formel (I) 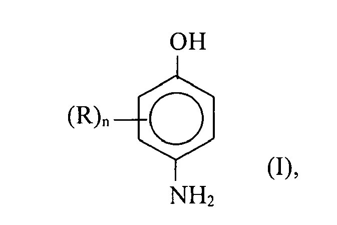 worin R eine C₁-C₃-Alkylgruppe, eine Hydroxy-C₁-C₃-Alkylgruppe oder ein Halogenatom, insbesondere ein Cl ist, und n eine Zahl von 0 bis 2, oder 2-Aminophenol oder 1,4 Diaminobenzene ist, oder substitutierte p-Phenylenediamine und deren Derivate mit der allgemeinen Formel II 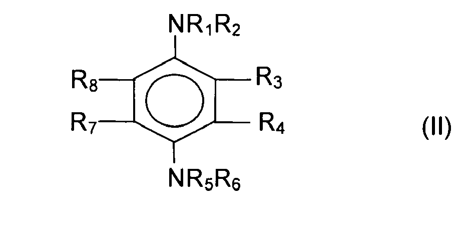 worin R₁ und R₂ gleich oder unterschiedlich sind, und für Wasserstoff, C₁-C₄ Alkyl, C₁-C₄ Hydroxyalkyl, Isopropyl, Phenyl oder p-Aminophenyl stehen, R₃ Wasserstoff, C₁-C₄ Alkyl, C₁-C₄ Hydroxyalkyl, C₁-C₄ Alkoxy, Chlorid oder Isopropyl ist, R₄ Wasserstoff, Halogen, insbesondere Chlorid, C₁-C₄ Alkyl oder C₁-C₄ Hydroxyalkyl ist, R₅ und R₆ gleich oder unterschiedlich, eine Gruppe ausgewählt aus Wasserstoff , C1-C4 Alkyl, C1 -C4 Hydroxyalkyl oder Isopropyl sind, R₇ Wasserstoff ist und R₈ eine Gruppe ausgewählt aus Wasserstoff, Hydroxyl, C₁-C₄ Alkyl, C₁-C₄ Hydroxyalkyl oder Isopropyl ist,
b- mindestens einen sauren direkt ziehenden Farbstoff, und
c- mindestens einen kationischen direkt ziehenden Farbstoff, ausgewählt aus Verbindungen, dargestellt mit den allgemeinen Formeln III, IV, V and VI 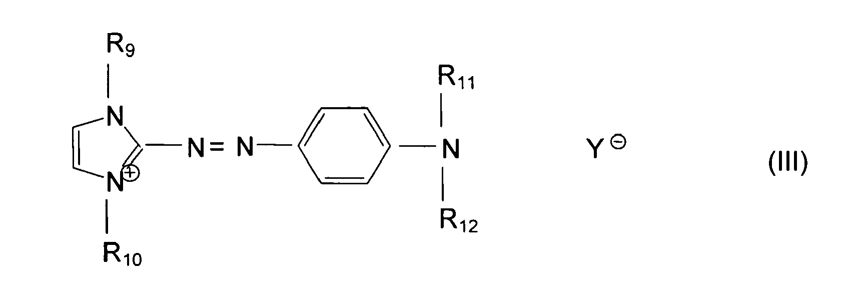 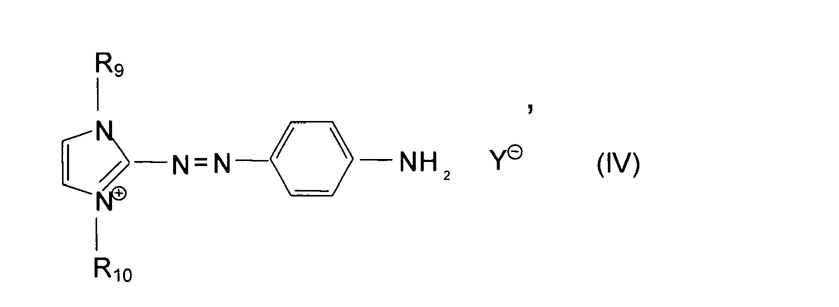 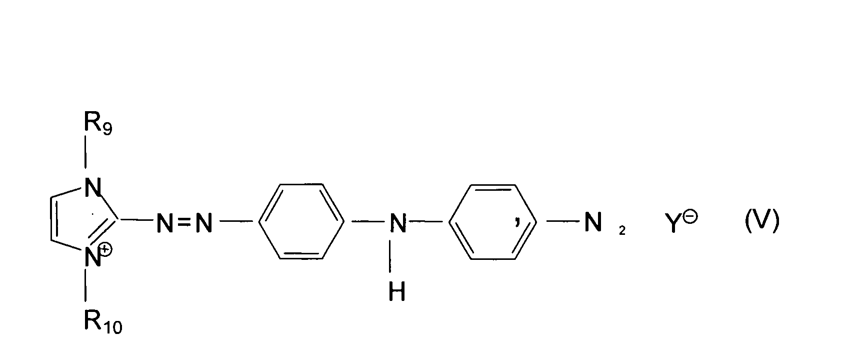 und 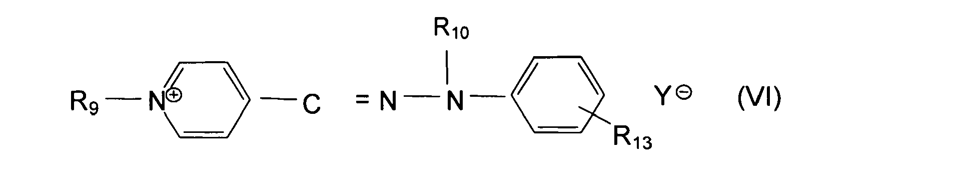
worin R₉, R₁₀, R₁₁ und R₁₂ für Wasserstofft, eine CH₃- oder C₂H₅- Gruppe stehen, R₁₃ für Wasserstoff, -OCH₃ oder -OC₂H₅ steht, und Y ein Anion wie Chlorid, Bromid, Methosulfat ist, enthält.

2. Zusammensetzung nach Anspruch 1, **dadurch gekennzeichnet, dass** sie als Komponente a mindestens einen Oxydationsfarbstoff- Precurser , ausgewählt aus den Verbindungen 4-Aminophenol, 4-Amino-3-methylphenol, 2-Chloro-4-aminophenol, 2.6-dichloro-4-aminophenol, 2.4-Diaminophenol, 2,6-Dibromo-4-aminophenol und/oder 2-Aminophenol, enthält.

3. Zusammensetzung nach Anspruch 1, **dadurch gekennzeichnet, dass** sie als Komponente mindestens einen Oxydationsfarbstoff- Precurser, ausgewählt aus den Verbindungen 2,5-Diamino-toluene, 2-n-Propyl or 2-ethyl-p-phenylene-diamine, 2,6-Dimethyl-p-phenylenediamine, 2-(2,5-Diaminophenyl) ethanol, 1-Amino -4-bis-(2'-hydroxy-ethyl)aminobenzene, 2-(2-Hydroxyethyl amino)-5-aminotoluene, 4,4'-Diaminodiphenylamine, 4-Aminodiphenylamine, 2-Amino-5-N,N-diethyl aminotoluene, 4-Amino-N-ethyl-N-isopropyl aniline, 2-Chloro-p-phenylenediamine, 1-β-Hydroxyethyl-2,5-diamino-4-chlorobenzene, 1-β-Hydroxyethyl-2,5-diamino-4-methyl benzene, 2-Methoxy-p-phenylenediamine, N,N-Diethyl-p-phenylenediamine, 1-Amino-4-β-methoxyethyl aminobenzene, 1-Dimethyl-amino-4-aminobenzene, 1-Hydroxy-2,5-diamino-4-methyl benzene, 1-Hydroxymethyl-2,5-diaminobenzene, 1.3-Dimethyl-2,5-diaminobenzene, 1.4-Diamino isopropyl benzene und/oder 1-Amino-4-β-hydroxypropyl aminobenzene oder deren wasserlösliche Salze, enthält.

4. Zusammensetzung nach einem der vorhergehenden Ansprüche, **dadurch gekennzeichnet, dass** sie als Komponente b mindestens einen sauren anionischen Farbstoff, ausgewählt aus den Verbindungen Acid Red 52, DC Violet 2, DC Red 33, DC Orange 4, DC Red 27 und DC Yellow 10, enthält.

5. Zusammensetzung nach einem der vorhergehenden Ansprüche, **dadurch gekennzeichnet, dass** sie als Komponente c den kationischen Farbstoff entsprechend der Formel III 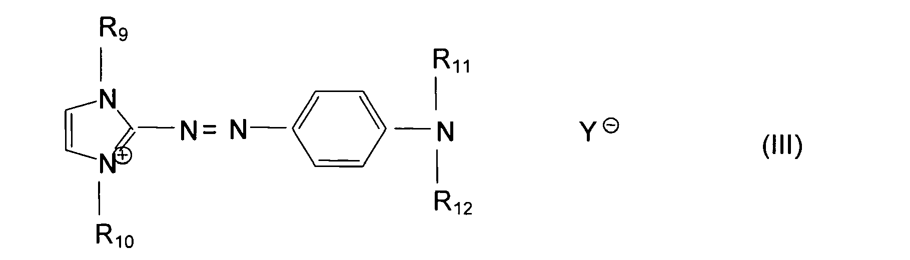 worin R₉, R₁₀, R₁₁ und R₁₂ Methyl sind und Y Chlorid ist, enthält.

6. Zusammensetzung nach den Ansprüchen 1 bis 4, **dadurch gekennzeichnet, dass** sie als Komponente c den kationischen Farbstoff entsprechend der Formel IV 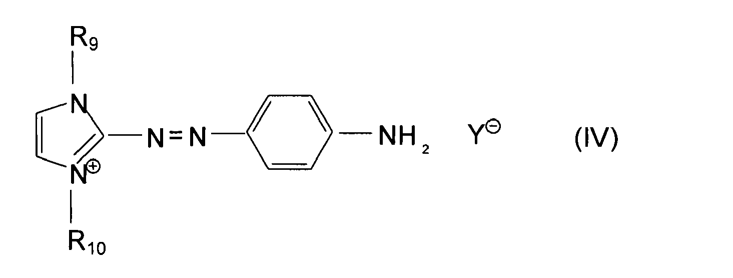 worin R₉, und R₁₀ Methyl sind und Y Chlorid ist, enthält.

7. Zusammensetzung nach den Ansprüchen 1 bis 4, **dadurch gekennzeichnet, dass** sie als Komponente c den kationischen Farbstoff entsprechend der Formel VI 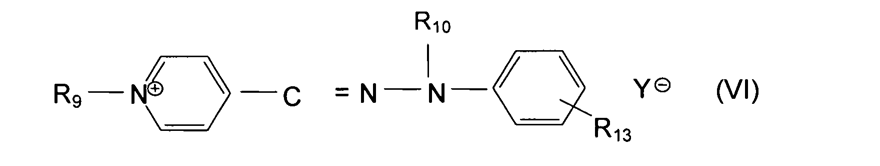 worin R₉ und R₁₀ Methyl sind, R₁₃ Wasserstoff ist und Y⁻ Methosulfat ist, enthält.

8. Zusammensetzung nach einem der vorhergehenden Ansprüche, **dadurch gekennzeichnet, dass** sie die Komponente a in einer Menge von 0,01 bis 5% Gew.- %, berechnet auf die Gesamtzusammensetzung, ausgenommen des Oxydationsmittels, enthält.

9. Zusammensetzung nach einem der vorhergehenden Ansprüche, **dadurch gekennzeichnet, dass** sie die Komponente b in einer Menge von 0,1 bis 7,5% Gew.- %, berechnet auf die Gesamtzusammensetzung, ausgenommen des Oxydationsmittels, enthält.

10. Zusammensetzung nach einem der vorhergehenden Ansprüche, **dadurch gekennzeichnet, dass** sie die Komponente c in einer Menge von 0,01 bis 2,5% Gew.- %, berechnet auf die Gesamtzusammensetzung, ausgenommen des Oxydationsmittels, enthält.

11. Zusammensetzung nach einem der vorhergehenden Ansprüche, **dadurch gekennzeichnet,dass** sie die Komponenten b und c in einem Gewichtsverhältnis von (kationischem Farbstoff : anionischem Farbstoff) 3:1 bis 1:10, vorzugsweise 2:1 bis 1:7, enthält.

12. Zusammensetzung nach einem der vorhergehenden Ansprüche, **dadurch gekennzeichnet, dass** sie zusätzlich mindestens eine Kupplersubstanz, ausgewählt aus Resorcinol, 2-Methyl resorcinol, 4-Chlororesorcinol, 2-Amino-4-chlorophenol, 5-Amino-4-methoxy-2-methylphenol, 3-Amino-phenol, 1-Methyl-2-hydroxy-4-aminobenzene, 3-N,N-Dimethyl aminophenol, 2,6-Dihydroxy-3,5-dimethoxypyridine, 5-Amino-3-methylphenol, 6-Amino-3-methylphenol, 3-Amino-2-methylamino-6-methoxypyridine, 2-Amino-3-hydroxy-pyridine, 2-Dimethyl-amino-5-aminopyridine, 2,6-Diaminopyridine, 1,3-Diaminobenzene, 1-Amino-3-(2'-hy-droxyethylamino)benzene, 1-Amino-3-[bis(2'-hydroxy-ethyl) amino]benzene, α-Naphthol, 4,6-Dichlororesorcinol, 1,3-Diaminotoluene, 1-Hydroxy naphthalene, 4-Hydroxy-1,2-methy-lenedioxy benzene, 1,5-Dihydroxy naphthalene, 1,6-Dihydroxy naphthalene, 1,7-Dihydroxy naphthalene, 2.,7-Dihydroxy naphthalene, 1-Hydroxy-2-methyl naphthalene, 4-Hydroxy-1,2-methyldioxy benzene, 2,4-Diamino-3-chlorophenol und/oder 1-Methoxy-2-amino-4-(2'-hydroxyethyl amino)benzene oder deren wasserlösliche Salze, enthält.

13. Zusammensetzung nach einem der vorhergehenden Ansprüche, **dadurch gekennzeichnet, dass** sie Organosiloxanpolymer entsprechend der Formel 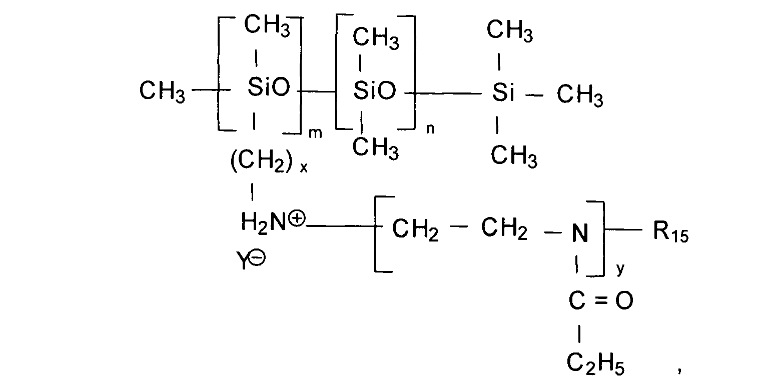 worin m und n jeweils Zahlen von 20 bis 10,000 sind, x eine Zahl zwischen 1 und 5 ist, und y eine Zahl von 5 bis 30 ist, R₁₅ eine C₁-C₁₂-Alkyl- oder Arylgruppe, im speziellen eine Methyl-, Ethyl- oder Benzylgruppe ist, and Y- ein Anion ist, enthält.

14. Zusammensetzung nach einem der vorhergehenden Ansprüche, **dadurch gekennzeichnet, dass** sie zusätzlich mindestens eine Ceramid-Verbindung entsprechend der Formel 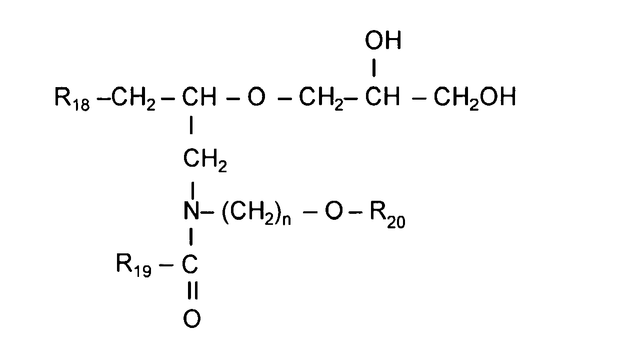 wobei R₁₈ und R₁₉ unabhängig voneinander Alkyl- oder Alkenylgruppen mit 10 bis 22 Kohlenstoffatomen sind, R₂₀ eine Methy-, Ethyl-, n-Propyl- oder Isopropylgruppe ist und n eine Zahl zwischen 1 bis 6, vorzugsweise 2 oder 3 ist, enthält.

15. Zusammensetzung nach einem der vorhergehenden Ansprüche, **dadurch gekennzeichnet, dass** sie mindestens ein Ubiquinone der Formel 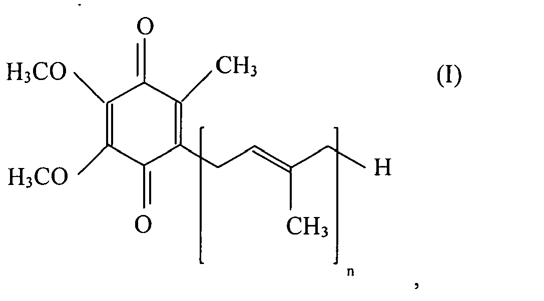 worin n eine Zahl von 1 bis 10 ist, enthält.

16. Zusammensetzung nach einem der vorhergehenden Ansprüche, **dadurch gekennzeichnet, dass** sie zusätzlich kationische und/oder neutrale HC Farbstoffe enthält.

17. Zusammensetzung nach einem der vorhergehenden Ansprüche ,**dadurch gekennzeichnet, dass** sie zusätzlich Kalium- oder Natriumjodid und/oder Dihydroxyaceton, als Katalysatoren, enthält.

18. Verwendung von Zusammensetzungen nach einem der vorhergehenden Ansprüche zum haltbaren Färben von Keratinfasern, insbesondere von Haaren.

## Revendications

1. Composition pour la coloration de fibres kératiniques, en particulier du cheveu humain, **caractérisée en ce qu'**elle comprend
a- au moins un précurseur de colorant d'oxydation, choisi parmi le 4-aminophénol et les dérivés de celui-ci de formule générale I 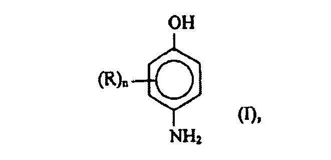 dans laquelle R représente un groupe alkyle en C₁-C₃, un groupe hydroxy-alkyle en C₁-C₃ ou un atome d'halogène, en particulier Cl, et n est un nombre de 0 à 2, ou le 2-aminophénol ou le 1,4-diaminobenzène ou les p-phénylènediamines substituées et les dérivés de celles-ci de formule générale II 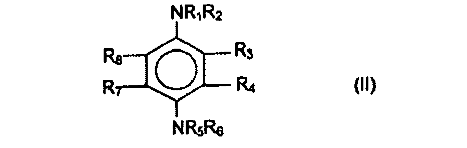 dans laquelle R₁ et R₂ sont identiques ou différents et représentent un hydrogène, un groupe alkyle en C₁-C₄, hydroxyalkyle en C₁-C₄, isopropyle, phényle ou p-amino-phényle, R₃ représente un hydrogène, un groupe alkyle en C₁-C₄, hydroxyalkyle en C₁-C₄, alcoxy en C₁-C₄, chloro ou isopropyle, R₄ représente un hydrogène, un halogène, en particulier le chlore, un groupe alkyle en C₁-C₄ ou hydroxyalkyle en C₁-C₄, R₅ et R₆ représentent un groupe identique ou différent choisi parmi l'hydrogène, les groupes alkyle en C₁-C₄, hydroxyalkyle en C₁-C₄ et isopropyle, R₇ représente un hydrogène et R₈ représente un groupe choisi parmi l'hydrogène, les groupes hydroxyle, alkyle en C₁-C₄, hydroxyalkyle en C₁-C₄ et isopropyle,
b- au moins un colorant direct acide, et
c- au moins un colorant direct cationique choisi parmi les composés représentés par les formules générales III, IV, V et VI 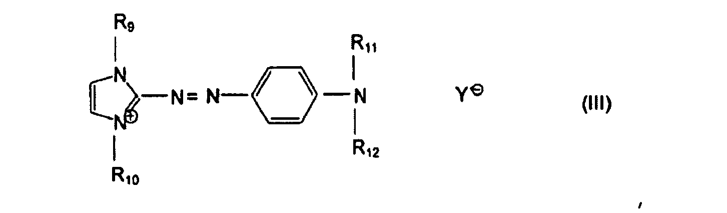 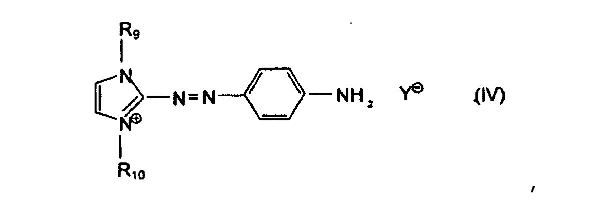 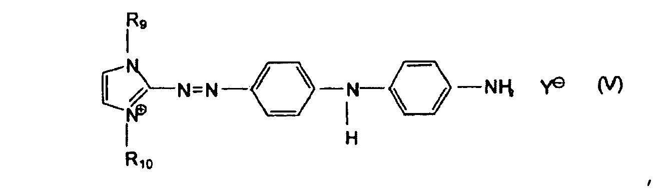 et 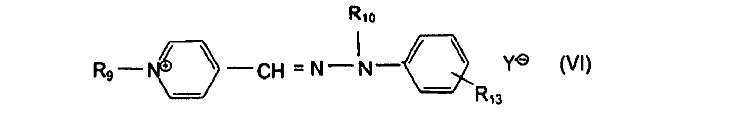
dans lesquelles
R₉, R₁₀, R₁₁ et R₁₂ représentent un hydrogène, un groupe CH₃- ou C₂H₅-, R₁₃ représente un hydrogène, -OCH₃ ou -OC₂H₅, et Y est un anion tel que le chlore, le brome, le méthosulfate.

2. Composition selon la revendication 1 **caractérisée en ce qu'**elle comprend comme composant a au moins un précurseur de colorant d'oxydation choisi parmi les composés 4-aminophénol, 4-amino-3-méthylphénol, 2-chloro-4-aminophénol, 2,6-dichloro-4-aminophénol, 2,4-diaminophénol, 2,6-dibromo-4-aminophénol et/ou 2-aminophénol.

3. Composition selon la revendication 1 **caractérisée en ce qu'**elle comprend comme composant a au moins un précurseur de colorant d'oxydation choisi parmi les composés 2,5-diamino-toluène, 2-n-propyl- ou 2-éthyl-p-phénylène-diamine, 2,6-diméthyl-p-phénylènediamine, 2-(2,5-diaminophényl)éthanol, 1-amino-4-bis-(2'-hydroxyéthyl)aminobenzène, 2-(2-hydroxyéthylamino)-5-aminotoluène, 4,4'-diaminodiphénylamine, 4-aminodiphénylamine, 2-amino-5-N,N-diéthylaminotoluène, 4-amino-N-éthyl-N-isopropylaniline, 2-chloro-p-phénylènediamine, 1-β-hydroxyéthyl-2,5-diamino-4-chlorobenzène, 1-β-hydroxyéthyl-2,5-diamino-4-méthylbenzène, 2-méthoxy-p-phénylènediamine, N,N-diéthyl-p-phénylènediamine, 1-amino-4-β-méthoxyéthylaminobenzène, 1-diméthyl-amino-4-aminobenzène, 1-hydroxy-2,5-diamino-4-méthylbenzène, 1-hydroxyméthyl-2,5-diaminobenzène, 1,3-diméthyl-2,5-diaminobenzène, 1,4-diaminoisopropylbenzène et/ou 1-amino-4-β-hydroxypropylaminobenzène, ou les sels hydrosolubles de ceux-ci.

4. Composition selon l'une quelconque des revendications précédentes **caractérisée en ce qu'**elle comprend comme composant b au moins un colorant anionique acide choisi parmi les composés Acid Red 52, DC Violet 2, DC Red 33, DC Orange 4, DC Red 27 et DC Yellow 10.

5. Composition selon l'une quelconque des revendications précédentes **caractérisée en ce qu'**elle comprend comme composant c le colorant cationique répondant à la formule III 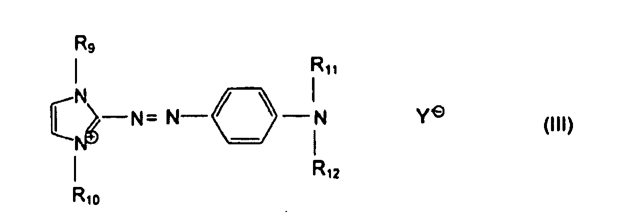 dans laquelle R₉, R₁₀, R₁₁ et R₁₂ représentent un groupe méthyle et Y est un chlorure.

6. Composition selon les revendications 1 à 4 **caractérisée en ce qu'**elle comprend comme composant c le colorant cationique répondant à la formule IV 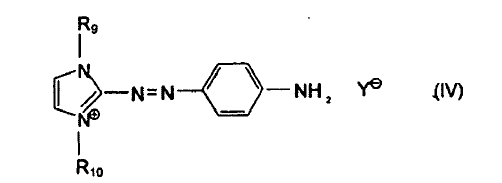 dans laquelle R₉ et R₁₀ représentent un groupe méthyle et Y est un chlorure.

7. Composition selon les revendications 1 à 4 **caractérisée en ce qu'**elle comprend comme composant c le colorant cationique répondant à la formule VI 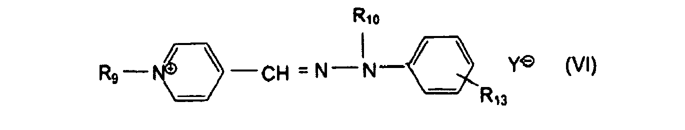 dans laquelle R₉ et R₁₀ représentent un groupe méthyle, R₁₃ est un hydrogène et Y représente un méthosulfate.

8. Composition selon l'une quelconque des revendications précédentes **caractérisée en ce qu'**elle comprend le composant a à une concentration de 0,01 à 5 % en poids calculée par rapport à la composition totale à l'exclusion de l'agent oxydant.

9. Composition selon l'une quelconque des revendications précédentes **caractérisée en ce qu'**elle comprend le composant b à une concentration de 0,1 à 7,5 % en poids calculée par rapport à la composition totale à l'exclusion de l'agent oxydant.

10. Composition selon l'une quelconque des revendications précédentes **caractérisée en ce qu'**elle comprend le composant c à une concentration de 0,01 à 2,5 % en poids calculée par rapport à la composition totale à l'exclusion de l'agent oxydant.

11. Composition selon l'une quelconque des revendications précédentes **caractérisée en ce qu'**elle comprend les composants b et c dans un rapport pondéral (colorant cationique : colorant anionique) de 3 : 1 à 1 : 10, de préférence 2 : 1 à 1 : 7.

12. Composition selon l'une quelconque des revendications précédentes **caractérisée en ce qu'**elle comprend en outre au moins une substance de couplage choisie parmi le résorcinol, le 2-méthylrésorcinol, le 4-chlororésorcinol, le 2-amino-4-chlorophénol, le 5-amino-4-méthoxy-2-méthylphénol, le 3-amino-phénol, le 1-méthyl-2-hydroxy-4-aminobenzène, le 3-N,N-diméthylaminophénol, la 2,6-dihydroxy-3,5-diméthoxypyridine, le 5-amino-3-méthylphénol, le 6-amino-3-méthylphénol, la 3-amino-2-méthylamino-6-méthoxypyridine, la 2-amino-3-hydroxypyridine, la 2-diméthyl-amino-5-aminopyridine, la 2,6-diaminopyridine, le 1,3-diaminobenzène, le 1-amino-3-(2'-hydroxyéthylamino)benzène, le 1-amino-3-[bis(2'-hydroxyéthyl)amino]benzène, le α-naphtol, le 4,6-dichlororésorcinol, le 1,3-diaminotoluène, le 1-hydroxynaphtalène, le 4-hydroxy-1,2-méthylènedioxybenzène, le 1,5-dihydroxynaphtalène, le 1,6-dihydroxynaphtalène, le 1,7-dihydroxynaphtalène, le 2,7-dihydroxynaphtalène, le 1-hydroxy-2-méthylnaphtalène, le 4-hydroxy-1,2-méthyldioxybenzène, le 2,4-diamino-3-chlorophénol et/ou le 1-méthoxy-2-amino-4-(2'-hydroxyéthylamino)benzène, ou les sels hydrosolubles de ceux-ci.

13. Composition selon l'une quelconque des revendications précédentes **caractérisée en ce qu'**elle comprend un polymère d'organosiloxane répondant à la formule 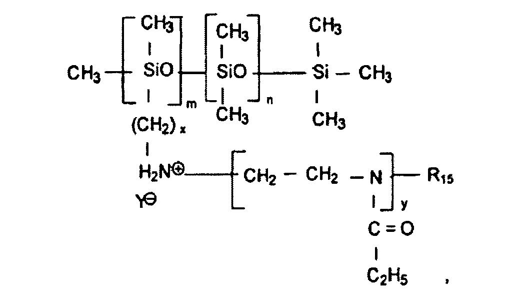 dans laquelle m et n sont tous deux des nombres de 20 à 10 000, x est un nombre entre 1 et 5, et y est un nombre de 5 à 30, R₁₅ représente un groupe alkyle ou aryle en C₁-C₁₂, en particulier un groupe méthyle, éthyle ou benzyle, et Y- est un anion.

14. Composition selon l'une quelconque des revendications précédentes **caractérisée en ce qu'**elle comprend en outre au moins un composé de type céramide répondant à la formule 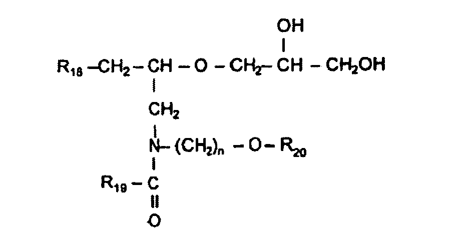 dans laquelle R₁₈ et R₁₉ représentent des groupes alkyle ou alcényle indépendants l'un de l'autre à 10 à 22 atomes de carbone, R₂₀ représente un groupe méthyle, éthyle, n-propyle ou isopropyle, et n est un nombre entre 1 et 6, de préférence 2 ou 3.

15. Composition selon l'une quelconque des revendications précédentes **caractérisée en ce qu'**elle comprend au moins une ubiquinone de formule 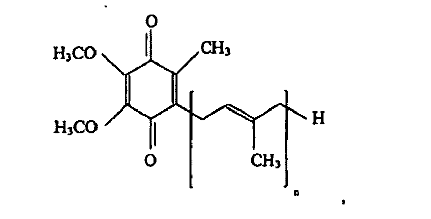 dans laquelle n est un nombre de 1 à 10.

16. Composition selon l'une quelconque des revendications précédentes **caractérisée en ce qu'**elle comprend des colorants HC cationiques et/ou neutres supplémentaires.

17. Composition selon l'une quelconque des revendications précédentes **caractérisée en ce qu'**elle comprend en outre de l'iodure de potassium ou de sodium et/ou de la dihydroxyacétone comme catalyseurs.

18. Utilisation de compositions selon l'une quelconque des revendications précédentes pour la coloration durable de fibres kératiniques, en particulier du cheveu.
